# EUROPEAN PATENT SPECIFICATION

(11) **EP 0 944 829 B1**
(45) Date of publication and mention of the grant of the patent: **02.07.2003**
(21) Application number: 97947812.0
(22) Date of filing: 10.12.1997
(51) Int. Cl.: G01N 33/50, C12Q 1/68

(54) **SELECTIVE LYSIS OF CELLS**
SELEKTIVES VERFAHREN ZUR LYSE VON ZELLEN
LYSE SELECTIVE DE CELLULES

(30) Priority: 11.12.1996 EP 96309016
(43) Date of publication of application: 29.09.1999
(73) Proprietor: Amersham plc, Little Chalfont Buckinghamshire HP7 9NA (GB)
(72) Inventor: RAYBUCK, Margaret, Amersham Buckinghamshire HP7 9LL (GB); SWEET, Alison, Amersham Buckinghamshire HP7 9LL (GB)
(74) Representative: Canning, Lewis R.
(86) International application number: GB9703402
(87) International publication number: WO98026284

(56) References cited:
- EP-A- 0 587 951
- WO-A-85/05684
- WO-A-94/17209
- WO-A-96/18878

## Description

The present invention relates to a method of obtaining cell nuclei of target nucleated cells which are foetal cells from a mixture of at least 2 different nucleated cell populations and to the subsequent analysis of such target cell nuclei. The method is useful in genetic analysis and diagnostic methods, such as prenatal diagnosis, cell purification or enrichment techniques for the elimination of one cell type, and therapeutic applications. The method is particularly useful for non-invasive prenatal diagnosis.

Current methods of prenatal diagnosis can be non-invasive such as biochemical screening of maternal serum or ultrasound; or may require foetal cells to be obtained using invasive techniques, such as amniocentesis or chorionic villus sampling (CVS). The non-invasive methods can provide only a limited amount of information about foetal abnormalities and invasive sampling methods carry a level of risk to both mother and foetus. Non-invasive approaches to diagnosis are desirable.

The detection of XY metaphases in the peripheral blood of pregnant women carrying male foetuses is proof of the passage of foetal cells into the maternal peripheral blood circulation. A number of different foetal cell types have been identified in maternal blood and which are potentially useful for prenatal diagnosis - trophoblast, lymphocyte, granulocyte and nucleated erythrocyte. All of these foetal cells are nucleated, i.e. they possess a cell nucleus which contains the full complement of foetal genes.

The isolation of foetal cells from maternal blood is difficult because they are present at extremely low levels - estimates range from 1 in 10⁵ to 1 in 10⁹ of the maternal blood cell population. Attempts to isolate foetal cells from maternal blood have been based on the enrichment of one particular cell type using a variety of methods. These approaches rely on specific characteristics of the target cells such as cell size, shape and surface antigenic markers to allow their differentiation from maternal cells. Methods have included density gradient centrifugation, antibody coated beads, fluorescence activated cell sorter (FACS) and magnetic activated cell sorter (MACS). Although density gradient centrifugation should provide a simple method of enriching a particular cell type, it has proved difficult in practice to achieve good recovery rates. Thus the selection of the cell layer which is most enriched for a particular cell type inevitably results in significant losses in other layers. The antibody-based approaches using FACS, MACS or immobilised antibodies have also proved difficult to give good purities due to the cross-reactivity of cell surface markers with other cell types. Although some of these methods have given good levels of enrichment, overall they are labour intensive with relatively poor recovery rates and low target cell purity.

To identify the cell as foetal, genetic analysis such as PCR (polymerase chain reaction) or FISH (fluorescent *in situ* hybridisation) has been used to detect foetal DNA sequences. The most definitive difference between mother and foetus is the presence of Y chromosome gene sequences when the foetus is male and hence the detection of male DNA sequences has been widely developed as a model system for confirmation of the foetal origin of cells.

A mammalian cell comprises a mass of cytoplasm separated from its environment by the plasma membrane. The structure of the plasma membrane varies between different cells depending on their physiological role, for example erythrocytes have a very uniform biconcave appearance facilitating their function as oxygen carriers in the blood. The mechanical stability and flexibility of the erythrocyte membrane comes from a partnership between the plasma membrane and the underlying meshwork of the cytoskeleton. Membranes are a complex organisation of macromolecules comprising a lipid bilayer embedded with proteins. The proportion of lipid, protein and other components such as cholesterol varies between different cell types depending on the function of the cell, for example in the erythrocyte the proportion of phospholipid and cholesterol in the plasma membrane is equimolar giving it a rigid but flexible structure which allows it to squeeze through narrow capillaries. Changes in the composition and fluidity of the plasma membrane, which can affect normal cell function, can be caused by a number of factors such as cell growth and differentiation, disease, hormone action and ageing. Eukaryotic cells also contain a large number of membranous organelles with specialised functions such as the nucleus which contains the DNA of the cell. The membranes of these intracellular organelles including the nuclear membrane also vary in their molecular composition enabling a diversity of metabolic functions within the cell. It is known that the plasma membrane contains a high percentage of cholesterol compared to internal membranes such as the nuclear envelope.

Detergents have been used to lyse a variety of cells including blood cells such as erythrocytes, lymphocytes and granulocytes. Detergent methods can be divided into two categories - those causing complete lysis of nucleated cells to release both plasma membrane and nuclear membrane contents, and those limiting lysis to disruption of the plasma membrane to generate intact cell nuclei. The type and concentration of detergent used determines the extent of cell lysis. Complete cell lysis has been used in a wide range of applications for isolating and studying cell components, particularly for the isolation of proteins and DNA. Nonidet™ P-40 (nonaethyleneglycol octylphenylether) and triple detergent buffers are commonly used to release proteins from cells, for example for immunoprecipitation. US 5389549 discloses that polyoxyethylene surfactants can be used to lyse erythrocytes and modify leucocytes for subsequent leucocyte classification. US 4668618 discloses a nuclear isolation medium containing a non-ionic surfactant for the lysis of the plasma membrane of mammalian cells whilst keeping the nuclear membrane intact. This medium is capable of lysing red blood cells but leaves white blood cell nuclei intact. US 5447864 discloses a method for isolating cell nuclei via selective lysis of the cell plasma membranes leaving the majority of the nuclear membranes intact. US 5447864 contrasts the disclosed method with prior art total cell lysis methods. Methods of lysing mature peripheral red blood cells are known and include saponin, detergents, osmotic lysis and a variety of other lytic reagents. Several buffers suitable for lysing red cells whilst maintaining the integrity of white cells are available commercially. US 4185964 discloses the use of a quaternary ammonium salt (e.g. cetyltrimethyl ammonium bromide ) to stromatolyse erythrocytes but not leucocytes for *in vitro* analysis of haemoglobin concentration. US 5258311 discloses that lithium salts can be used to lyse erythrocytes resulting in the release and denaturation of haemoglobin for immunoassay of haemoglobin concentration.

Saponin (a plant-derived glycoside) is well known for lysing adult/mature red blood cells but when present in excess can also damage leucocytes. Saponin has also been used to permeabilise but not solubilise the plasma membrane of rat hepatocytes whilst retaining the integrity of internal membranes in order to study the release of secretory proteins. Saponin permeabilisation has been used to allow entry of macromolecules into the cell whilst maintaining overall cell integrity. US 4751179 discloses that a formic acid based lytic reagent which also contains saponin and a cross-linking agent can be used to selectively lyse red cells and differentially modify white cells allowing the leucocyte sub-populations to be identified by flow cytometry. Saponin was added to reduce the size of the red cell fragments to minimise interference with the analysis. US 5232857 discloses that a combination of saponin and sodium dodecylsulphate (SDS) can be used to lyse erythrocytes and solubilise the leucocyte plasma membrane whilst differentially staining the leucocyte sub-populations for analysis by flow cytometry.

The mechanism of osmotic lysis of erythrocytes involves net water flux into the cell due to either low external osmolality (hypotonic lysis) or changes in membrane permeability to osmolytes (colloid osmotic lysis). The resulting osmotic pressure can be sufficient to cause lysis. Hypotonic lysis relies on using a buffer with an appropriate concentration of an inorganic salt (such as sodium chloride or ammonium chloride) to cause the swelling of erythrocytes such that they burst. White cells are known to be more stable to changes in the ionic strength of the surrounding medium than red cells. Aqueous sodium chloride is well known for causing osmotic lysis and has been used in the osmotic fragility test as a diagnostic tool for red cell membrane defects. The use of ammonium ions and carbon dioxide for hypotonic lysis of red blood cells was first published in the 1930's. This method has since been used in a number of applications as an alternative to density gradient centrifugation to separate out nucleated cells from whole blood. Ammonium chloride induced lysis has been used to preferentially eliminate red blood cells prior to FACS analysis.

US 5516695 discloses a multipurpose reagent suitable for the automated differentiation of white blood cells and detection of nucleated red blood cells (NRBC) which concurrently lyses the red blood cells and fixes the white blood cells. The reagent comprises:
(i) a non-quaternary ammonium salt,
(ii) a C₁₋₄ aliphatic aldehyde,
(iii) buffers,
and optionally (iv) 10-200 mg/L of a surface active agent to preserve
white blood cell membranes.
The function of the aldehyde is to fix the white blood cells.

US 5516695 also discloses that, when NRBC's are to be detected, then a nuclear stain such as ethidium homodimer is added to the reagent before the lytic reagent is added to the blood sample.

US 5559037 discloses a method of differentiating nucleated red blood cells from other cells by flow cytometry following treatment of samples of whole blood with the multipurpose reagent of US 5516695.

Studies on foetal erythrocytes from cord blood have shown that they differ from adult erythrocytes in a number of ways, including their water permeability, bicarbonate-chloride exchange rate and plasma membrane lipid composition. Hypotonic lysis with carbonic anhydrase and NH₄Cl-HCO₃ has been used to preferentially lyse maternal red cells in the presence of foetal red cells [S H Boyer *et al,* Blood, 47, 883(1976)]. This method utilises the differences in the activity of the enzyme carbonic anhydrase within maternal and foetal cells to selectively lyse maternal red cells whilst the foetal red cells remain intact. Foetal blood from the umbilical cord has been reported to contain two distinct red cell populations - a minority of rapidly lysed cells with carbonic anhydrase levels similar to adult cells and a majority of slowly disrupted cells in which the concentration of carbonic anhydrase is significantly lower than in adults. Studies on the differences in osmotic fragility between human foetal (umbilical cord) and adult red blood cells have also found that there appears to be a subpopulation of cells in cord blood highly resistant to lysis which are believed to be young cells. The principle of the Boyer lysis method has been used in a number of ways as part of an enrichment process to remove bulk maternal red cells from maternal samples prior to foetal cell isolation. WO 94/17209 discloses that ammonium chloride can be used to separate foetal nucleated cells from maternal non-nucleated red cells prior to depletion of white cells using a membrane filtration device. Intact foetal nucleated red cells pass through a filter whilst white cells are retained on the filter. Ammonium chloride based selective lysis has also been used to remove non-nucleated cells and produce an enriched nucleated cell sample for stimulation of growth of foetal erythroid progenitor cells with erythropoietin in culture. WO 95/03431 discloses that potassium chloride can be used in a preliminary selective lysis step to remove maternal red blood cells prior to further analysis by FACS or MACS.

### Description of the Invention

The present invention provides a method as defined in claim 1 of obtaining cell nuclei of target nucleated cells which are foetal cells from a mixture of at least two different nucleated cell populations, which comprises treating said mixture with a lytic reagent so as to selectively lyse the plasma membranes but not the nuclear membranes of the target nucleated cells whilst leaving the other nucleated cells intact. The method of the present invention comprises the steps of mixing the cell sample with a lytic reagent, performing an incubation and, preferably, isolating the nuclei. The method may be used to obtain isolated nuclei from the target cell for analysis or may be used to enrich a cell population in a mixed cell sample by selectively eliminating one cell type. The isolated foetal target nuclei obtained by the method may be either retained for further analysis or discarded whilst retaining the remaining intact nucleated cells. The foetal cells are of interest as potential sources of diagnostic or genetic information. The target foetal nucleated cells may be present in the mixture to be analysed as a very low proportion of the total cell population, i.e. be rare cells. Examples of such rare cells are foetal cells in maternal peripheral blood. The lysis method permits the selective liberation of the cell nuclei of target cells in the presence of a variety of other cells, including other nucleated cells. In particular, the selective lysis method lyses the plasma membranes of the target cells whilst leaving the nuclear membranes of the target cells and the plasma membranes of the non-target nucleated cells intact. Once liberated, the target cell nuclei can be analysed *in situ* or, preferably, are first isolated and (optionally) purified prior to analysis. The isolated nuclei can be analysed to identify the cellular origin of the nuclei and to obtain information about the genetic content of the cell. The cellular origin of the nuclei may be confirmed using markers specific to the nuclei of the target cell including nuclear membrane markers, cytoskeletal markers, mRNA or DNA and protein by such methods as *in situ* hybridisation, *in situ* amplification and *in situ* immunocytochemistry. The foetal origin of the liberated nuclei can be confirmed by analysis using foetal cell proteins or mRNA transcripts of their genes such as foetal haemoglobin (HbF), a-feto protein (AFP), erythropoietin, transferrin, human chorionic gonadotrophin, placental lactogen, or foetal DNA such as HLA (human leukocyte antigen) sequences, sex chromosomes X and Y, or foetal nuclear membrane surface markers or a combination of these markers. Analysis of the nucleic acid sequences (DNA and RNA) within the target cell nuclei can be carried out by PCR, FISH, DNA sequencing or other genetic profiling or mutation analysis methods known to those skilled in the art.

The term "nucleated cell" means a mammalian cell which possesses a cell nucleus. The term "rare cells" means cells which are present in the sample to be analysed as a low proportion of the total cell population (typically from 1 in 10⁴ to 1 in 10¹⁰). The total cell population will contain a variety of other nucleated and non-nucleated cells which are different to the rare cells. Examples of rare cells are foetal cells in maternal peripheral blood. The term "peripheral blood" means whole blood drawn from the peripheral blood circulation (e.g. arm vein) of a mammal. The term "foetal cells" means cells originating from the foetus including nucleated red blood cells and leucocytes. The term "cord blood" means blood drawn from the umbilical cord of a neonate at term delivery. The term "substantially intact' means that 90-100%, preferably 95-100% and most preferably 99-100% of the non-target cells are left intact. During foetal development a proportion of the peripheral foetal red blood cells have a cell nucleus and are termed immature red blood cells or erythroblasts. Erythroblasts are thus classified as nucleated cells and have both plasma and nuclear cell membranes. In contrast, peripheral adult red blood cells have no cell nucleus and hence have only a plasma membrane. It is the cell nucleus which contains the full complement of genes and is useful for genetic analysis.

The sample to be analysed may be maternal peripheral blood, foetal blood, cord blood, amniotic fluid, transcervical washings or a tissue sample (e.g. *via* chorionic villus sampling), preferably maternal peripheral blood. When the sample to be used is peripheral blood, the method may optionally include treatment of the blood prior to analysis with an anticoagulant such as ACD (acid citrate dextrose) or heparin. For maternal peripheral blood the sample is typically 5-50 ml of whole blood. The sample for foetal diagnosis can be taken anytime in the first 4 months of pregnancy, preferably at 6-16 weeks gestation. When the sample to be used is a tissue sample the method may optionally include treatment of the sample prior to analysis to homogenise the tissue to produce a dispersed mixture of cells. It is envisaged that the sample to be used in the present invention can be used directly (i.e. without any artificial enrichment) or pre-enriched to increase the proportion of target cells present in the sample. The sample may optionally be pre-treated to pre-enrich for the foetal target cell type. The sample may be cultured prior to lysis in order to increase the proportion of target cells in the mixture. The proportion of foetal cells in the sample may be pre-enriched by culturing the cells to stimulate growth of the foetal cells, for example by culturing in the presence of erythropoietin. The target nucleated cells may be pre-enriched by density gradient centrifugation, for example using Ficoll™. The sample may be pre-enriched for the target nucleated cells by agglutination with an antibody which cross-reacts with the cell surface of the target cells forming a complex of agglutinated target cells which can then be isolated by centrifugation ready for selective lysis. Alternatively, the sample can be pre-treated with an antibody which cross-reacts with the non-target cells resulting in a complex of agglutinated unwanted cells which can then be isolated by centrifugation and may be discarded. When the target cell is a foetal erythroblast, complexes of mature and immature red blood cells (including foetal erythroblasts) can be produced by agglutination using an antibody specific to an erythrocyte cell surface marker. The sample may be optionally pre-treated with reagents to enhance the selectivity of the lysis step. The efficiency of selectivity of the lysis may be enhanced by pre-treating the cells with antibodies which bind to the cell surface of the target cell or the non-target cell altering their susceptibility to lysis. The pre-treatment may increase the susceptibility of the target cells to lysis or alternatively decrease the fragility of the non-target cells. The pre-treatment may alter the lipid and cholesterol characteristics of the different cell membranes.

The selective lysis of the present invention is performed using a lysis solution composed of a mild lytic reagent and a (buffer) salt concentration to produce a mild osmotic shock. The lytic reagent used in the method suitably comprises one or a combination of the following reagents: a plant glycoside such as saponin or digitonin; a detergent, such as Triton X-100 (Triton is a registered trademark of Union Carbide Chemicals and Plastics Co. Inc.), Triton X-45, or sodium dodecylsulphate (SDS) or an inorganic salt such as an alkali metal salt (e.g. sodium chloride) or an ammonium salt (e.g. ammonium chloride). Preferred lytic reagents are plant glycosides such as saponin or non-ionic surfactants such as the Triton series. Most preferred are plant glycosides, especially saponin.

The selective lysis method of the present invention comprises the steps of mixing an aliquot of the sample with a volume lysing reagent and incubating. The selective lysis step should be performed in the temperature range 4 - 50°C and is preferably performed at ambient temperature (16 - 25°C). The time required for incubation of the sample with the selective lysis reagent is in the range 1 minute to 60 minutes and is preferably performed for 10 - 15 minutes.

The lytic reagent is dissolved or dispersed in either water or an organic solvent or mixtures thereof, where the organic solvent is suitably chosen from methanol, ethanol or DMSO (dimethylsulphoxide). The preferred medium is water and the pH of the solution should be in the range 6-8, preferably pH 7 to 7.5. The lysis solution may optionally contain additional reagents such as: reagents to protect the liberated target cell nuclei (suitably chosen from e.g. divalent cations, polyamines or sucrose); reagents to inhibit lysis of non-target nucleated cells (suitably chosen from e.g. polycarboxylic acids or osmotically active agents such as dextran); preservatives to inhibit growth of micro-organisms (suitably chosen from e.g. azide or Kathon™, i.e. octhilinone); reagents for enhancing isolation of nuclei (such as divalent cations); and viscosity reagents (suitably chosen from e.g. glycerol, polyethylene glycol). Preferred lysis solutions comprise a plant glycoside, especially saponin as the lytic reagent in aqueous medium. The aqueous solution also preferably contains buffers to maintain the pH in the range 7 to 7.5 and inorganic salts, especially alkali metal salts at a concentration of 10 to 100 mM. A most preferred lysis solution uses 0.01-0.1% (w/v) aqueous saponin in a mixture of:
60mM sodium chloride,
4.4mM phosphate buffer (pH 7.4),
1.2mM potassium chloride.

Once the lysis incubation is complete a quenching reagent may be added to prevent further lysis occurring. Preferred quenching reagents are physiologically buffered salt solutions, such as phosphate buffered saline (PBS). Such buffered salt solutions restore the osmotic balance of the remaining cells preventing further lysis.

The liberated target cell nuclei are isolated and purified to remove any contaminating intact cells and cell debris. The cell nuclei can be isolated by capture onto a membrane, density gradient centrifugation, binding to immobilised antibodies, fluorescence activated cell sorting or other methods known to those skilled in the art.

The liberated target cell nuclei of the present invention can be analysed as intact cell nuclei, or first lysed to release the target cell nuclei contents. When the isolated cell nuclei are maintained intact they can be used in a variety of applications such as studies of the mechanism of transcription, *in situ* hybridisation etc. When the target cell nuclei have been isolated by capture on a membrane, such as with Nuclitips™, the intact cell nuclei may be analysed directly whilst still attached to the membrane filter or released from the membrane intact for further analysis. The intact cell nuclei may be analysed by such methods as *in situ* hybridisation, *in situ* amplification or *in situ* immunocytochemistry or other methods known to those skilled in the art, to detect nucleic acids, nuclear membrane markers , mRNA transcripts, or proteins. The nuclear membrane of the liberated target cell nuclei can also be lysed prior to analysis using detergent or other methods known to those skilled in the art, to release the contents of the target cell nuclei, including the DNA. The nuclear extract produced may be used in a variety of applications, including *in vitro* transcription, characterisation of nuclear proteins etc. When the nuclei are lysed to release the nucleic acids, the DNA or RNA may be purified prior to analysis. Analysis may include such methods as PCR, DNA sequencing and other gene profiling and mutation analysis methods known to those skilled in the art. The method of the invention according to claim 9 is useful to analyse the nucleic acids for particular DNA sequences such as the sequence of a gene associated with a disease causing mutation, or sequences for gender prediction or chromosome abnormalities.

The present method shows that the selective lysis of the plasma membrane of foetal cells to generate intact foetal cell nuclei, whilst leaving maternal nucleated blood cells intact is possible. This is in contrast to prior art methods of maternal-foetal selective cell lysis which rely on maintaining the foetal cells completely intact and lysing (and removing) the maternal peripheral red blood cells as a means of enriching the foetal cell concentration. The prior art also teaches away from the present invention in suggesting that young foetal cells are more resistant to lysis than maternal cells. Prior art reagents for the isolation of cell nuclear contents show selectivity between the cell plasma and nuclear membranes, but there is no differentiation in terms of cell type - the methods are relatively harsh and result in the indiscriminate lysis of all nucleated cells present in a mixed population. Prior art selective lysis methods relate to the lysis of non-nucleated cells especially erythrocytes in the presence of nucleated cells such as leucocytes. In contrast, the present method describes the selective lysis of the plasma membrane of target nucleated cells, but not other nucleated cells in a mixture of nucleated cells.

The present lysis method permits the selective liberation of foetal cell nuclei from samples containing both foetal and maternal blood cells including, but not limited to, the situation when the foetal cell is present as a rare cell such as in maternal peripheral blood. The invention thus provides a method of generating foetal cell nuclei from such mixed maternal/foetal cell samples with minimal contamination from maternal cell nuclei. The lysis method of the present invention is useful in prenatal diagnosis for foetal abnormalities such as genetic disorders (e.g. β-globin mutations or cystic fibrosis) or chromosomal disorders such as Klinefelter syndrome or aneuploidies (e.g. Down's syndrome). For prenatal screening to be commercially feasible and to allow its availability to every pregnant woman (not just those considered at risk of foetal abnormality), it must be economically and technically efficient. Each stage must be safe and simple and easy to use. There is also a need for rapid sample processing to allow early clinical decisions. The method of the present invention provides a safe, simple, rapid and non-invasive means to obtain foetal cell nuclei for diagnosis. The method has the advantage over prior art methods that the diagnosis can be carried out on samples (such as maternal peripheral blood) in which the foetal cell population is a very small fraction of the total cell count without the requirement for cell enrichment methods. The method has the additional advantage that specialised reagents (such as monoclonal antibodies or density gradients), or expensive instrumentation (such as a flow cytometer) are not required. It is envisaged that the present method can be used as the first stage of a diagnostic method in its own right, or as an aid to early prenatal diagnosis prior to confirmation by standard invasive methods. It is envisaged that the method of the present invention could form part of a kit for sample preparation for diagnostic analysis and may contain a combination of reagents for performing the lysis step, nuclei isolation and analysis.

When the foetal target nucleated cells are for prenatal diagnosis, the present method does not need to distinguish between the different foetal cell types. Selective lysis of the plasma membrane of any or all of the nucleated foetal cells is useful in releasing foetal cell nuclei for analysis. The present lysis method selectively lyses foetal cells and maternal red blood cells (erythrocytes) in the presence of nucleated maternal peripheral blood cells. Thus, although maternal erythrocytes may be lysed, no cell nuclei of maternal origin are generated because mature red blood cells such as those from adult peripheral blood do not have a cell nucleus. Secondly, the method selectively lyses the foetal cells so that the plasma membrane of the majority of the foetal cells is lysed whilst the nuclear membrane remains largely intact. This results in a mixed population of intact maternal nucleated blood cells, foetal cell nuclei and cell debris. The foetal cell nuclei are then isolated and purified to remove any contaminating intact cells and cell debris. The foetal cell nuclei can be isolated by capture onto a membrane, density gradient centrifugation, binding to immobilised antibodies, fluorescence activated cell sorting or other methods known to those skilled in the art. A preferred method of isolation is specific capture onto a membrane filter using Nuclitips™ (the Trademark of a product marketed by Amersham International plc and which is described in US 5447864). Once captured on the Nuclitips™ membrane, the foetal cell nuclei can be readily purified by washing steps (to remove intact cells and cell debris) followed by analysis.

Examples one and two show the application of the method of the present invention to the selective lysis of foetal nucleated cells in the presence of adult blood cells. The sample is a mixture of male cord blood cells (foetal) and female adult blood cells.

### Example One

Blood (ca.35ml) was drawn from the arm of a non-pregnant female into a tube containing anticoagulant (ACD). Cord blood (ca.7ml) was obtained at term from the umbilical cord of a male baby and mixed with anticoagulant (heparin). Adult blood (0.5ml) was spiked with 1, 10 or 100 µl of cord blood by mixing. Samples (0.5ml) of adult, cord and adult-cord mixtures were treated separately for 10 minutes at room temperature with each of the following:
i) selective lysis 4.5 ml of Selective Lysis Buffer (0.055% saponin [quillaja bark], 4.44mM sodium phosphate buffer, 1.2mM KCl, 60.89 mM NaCl),
ii) detergent lysis of the plasma membrane of all nucleated cells 0.5ml of Lysis Buffer 1 (320mM sucrose, 10mM Tris pH 8, 5mM MgCl₂, 1% Triton X-100),
iii) no lysis addition of phosphate-buffered saline (4.5 ml 10mM sodium phosphate buffer, 2.7mM KCl, 137mM NaCl).

The nuclei were isolated using Nuclitips™ and the membrane-bound nuclei washed with phosphate buffered saline (PBS) to remove cell debris and other contaminants. The nuclei were lysed to release the DNA by addition of Nuclear Lysis Buffer 2
(20mM Tris(hydroxymethyl)aminomethane pH 8, 1mM EDTA, 0.2% SDS, 0.2mg/ml RNAse A, 2U/ml RNAse T1, 1mg/ml proteinase K) and incubated at 55°C for 30 minutes followed by 10 minutes at 80°C. The DNA was eluted from the Nuclitip by spinning briefly in a microfuge at 13000rpm. An aliquot of the extracted DNA was subjected to PCR using primers specific to (a) Y-chromosome sequences according to Lo *et al* [Lancet, 335, 1463 (1990)], and (b) a region close to the cystic fibrosis mutation on chromosome 7 according to Huth *et al* [Nucl.Acid Res., 17, 7118, (1989)] (this will amplify DNA from male or female). The PCR products were analysed by agarose gel electrophoresis and visualised by staining the DNA with ethidium bromide.

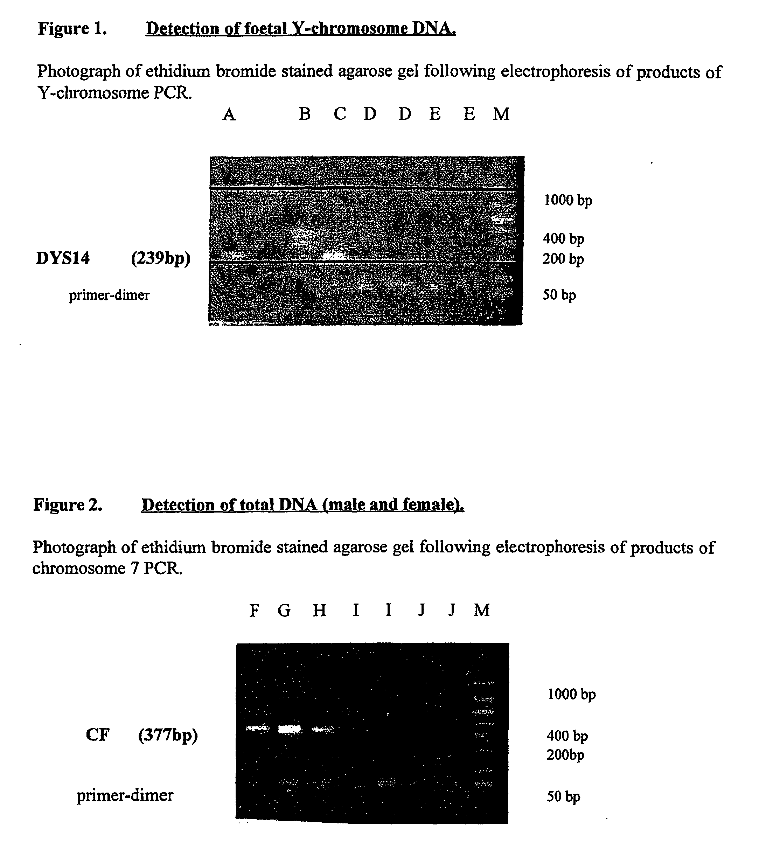

Reference is directed to the accompanying drawings, in which:-
Figure 1 is a photograph of ethidium bromide stained agarose gel following electrophoresis of products of Y-chromosome PCR. The lanes are identified as follows.
Lysis of all nucleated cells in sample using Lysis Buffer 1:

| | Result |
|---|---|
| A = 0.5 ml male cord blood | + |
| B = 0.5 ml female adult blood | - |
| Selective lysis of foetal nucleated cells in sample using Selective Lysis Buffer: | |
| C = 0.5 ml female adult blood spiked with 100 µl male cord blood | + |
| D = 0.5 ml female adult blood spiked with 10 µl male cord blood | - |
| E = 0.5 ml female adult blood | - |
| M = PCR molecular weight marker 50 - 1000bp. | |

Figure 2 is a photograph of ethidium bromide stained agarose gel following electrophoresis of products of chromosome 7 PCR.
Lysis of all nucleated cells in sample using Lysis buffer 1:

| | Result |
|---|---|
| F = 0.5 ml male cord blood | + |
| G = 0.5 ml female adult blood | + |
| Selective lysis of foetal nucleated cells in sample using Selective Lysis Buffer: | |
| H = 0.5 ml female adult blood spiked with 100 µl male cord blood | + |
| I = 0.5 ml female adult blood spiked with 10 µl male cord blood | - |
| J = 0.5 ml female adult blood | - |
| M = PCR molecular weight marker 50 - 1000bp. | |

Figure 1 shows that using the selective lysis buffer on the foetal I adult mixture followed by amplification of male sequences a specific Y-chromosome signal is visible; however, using the same lysis conditions on female adult blood no signal is visible. Figure 2 shows that using the selective lysis buffer on the foetal / adult mixture followed by amplification of male and female DNA a specific chromosome 7 signal is visible; however, using the same selective lysis conditions on female adult blood no signal is visible. This confirms that the DNA isolated from the foetal / adult mixture is from lysis of foetal nucleated cells and that there is no contamination from female adult DNA due to lysis of adult nucleated cells. In Figure 2 comparison of the two lysis buffers also demonstrates the difference in selectivity with the Selective Lysis Buffer - when female adult blood is treated with Selective Lysis Buffer no PCR signal is visible but female adult blood treated with the Lysis Buffer 1 gives a positive PCR signal. Thus, the Selective Lysis Buffer has a further level of differentiation enabling selective lysis of foetal but not adult nucleated cells whereas Lysis Buffer 1 lyses all nucleated cells.

### Example Two

Blood (ca.35ml) was drawn from the arm of a non-pregnant female adult and a male adult into separate tubes containing anticoagulant (ACD). Cord blood (ca.7ml) was obtained at term from the umbilical cord of a male baby and mixed with anticoagulant (heparin). Female adult blood was spiked with 0.1, 1, 10 or 100 µl of cord blood by mixing to give a total sample volume of 0.5ml. Samples (0.5ml) of female and male adult, cord and adult-cord mixtures were treated separately for 10 minutes at room temperature with each of the following:
i) selective lysis 4.5 ml of Selective Lysis Buffer (0.055% saponin, 3.33mM sodium phosphate buffer, 0.9mM KCI, 45.7mM NaCl),
ii) detergent lysis of the plasma membrane of all nucleated cells 0.5ml of Lysis Buffer 1 (320mM sucrose, 10mM Tris pH 8, 5mM MgCl₂, 1% Triton X-100),
iii) no lysis addition of 4.5 ml phosphate-buffered saline (10mM sodium phosphate buffer, 2.7mM KCI, 137mM NaCI).

The nuclei were isolated using Nuclitips™ and the DNA extracted as described in Example One. An aliquot of the extracted DNA was subjected to PCR as described in Example One. The PCR products were analysed by agarose gel electrophoresis and visualised by staining the DNA with Vistra Green.

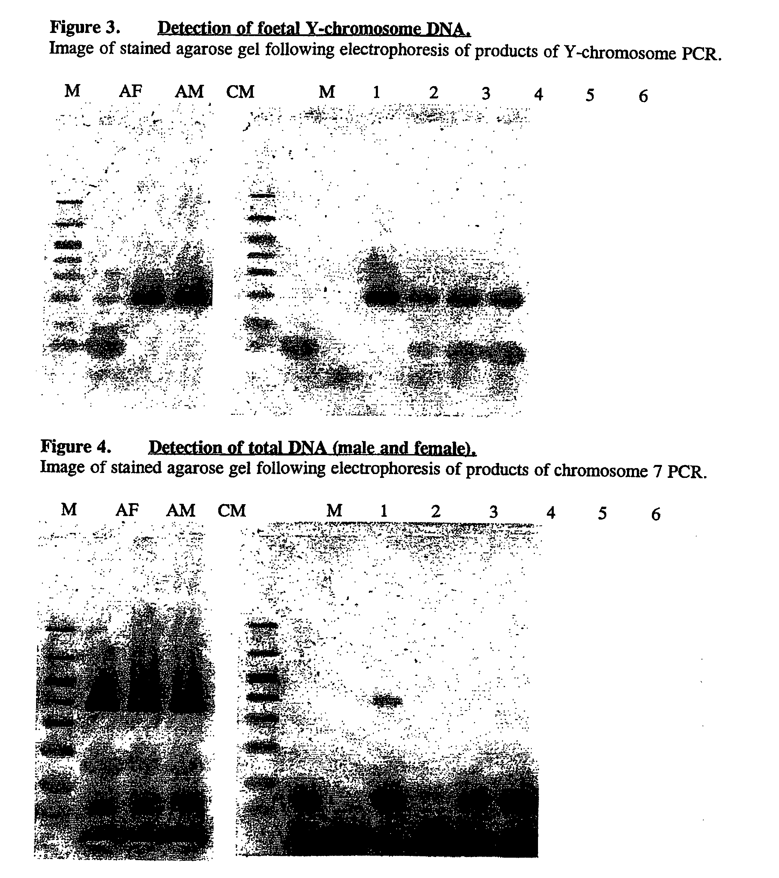

Figure 3 is an image of the stained gel following electrophoresis of the products of Y-chromosome PCR for the detection of male DNA sequences only. The lanes are identified as follows :
Lysis of all nucleated cells in sample using lysis buffer 1 :

| | Result |
|---|---|
| AF = 0.5ml female adult blood | -(non-specific bands) |
| AM = 0.5ml male adult blood | + |
| CM = 0.25ml male cord blood | + |

Selective lysis of foetal nucleated cells in sample using Selective Lysis Buffer:

| | Result |
|---|---|
| 1 = 0.5ml female adult blood | - |
| 2 = 0.5ml male adult blood | - |
| 3 = 0.4ml female adult blood spiked with 100ml male cord blood | + |
| 4 = 0.49ml female adult blood spiked with 10ml male cord blood | + |
| 5 = 0.5ml female adult blood spiked with 1ml male cord blood | + |
| 6 = 0.5ml female adult blood spiked with 0.1ml male cord blood | + |
| M = PCR molecular weight marker 50 - 1000bp | |

Figure 4 is an image of the stained gel following electrophoresis of the products of chromosome 7 PCR for the detection of male and female DNA sequences. The lanes are identified as follows :
Lysis of all nucleated cells in sample using lysis buffer 1:

| | Result |
|---|---|
| AF = 0.5ml female adult blood | + |
| AM = 0.5ml male adult blood | + |
| CM = 0.25ml male cord blood | + |
| | |

Selective lysis of foetal nucleated cells in sample using Selective Lysis Buffer:

| | |
|---|---|
| 1 = 0.5ml female adult blood | - |
| 2 = 0.5ml male adult blood | - |
| 3 = 0.4ml female adult blood spiked with 100ml male cord blood | + |
| 4 = 0.49ml female adult blood spiked with 10ml male cord blood | - |
| 5 = 0.5ml female adult blood spiked with 1ml male cord blood | - |
| 6 = 0.5ml female adult blood spiked with 0.1ml male cord blood | - |
| M = PCR molecular weight marker 50 - 1000bp | |

Figure 3 shows that using the selective lysis buffer on the foetal I adult mixture followed by amplification of male sequences a specific Y-chromosome signal is visible with a 0.1ml cord spike; however, using the selective lysis conditions on female or male adult blood no signal is visible.

Figure 4 shows that using the selective lysis buffer on the foetal / adult mixture followed by amplification of male and female DNA a specific chromosome 7 signal is visible with the 100ml spike; however, using the same selective lysis conditions on female adult or male adult blood no signal is visible. A positive signal is not observed with the lower cord spikes due to the lower sensitivity of the chromosome 7 PCR system. This confirms that the DNA isolated from the foetal / adult mixture is from lysis of male foetal nucleated cells and that there is no detectable contamination from female adult DNA due to lysis of adult nucleated cells. These results show that selective lysis of foetal nucleated cells in a mixture with adult cells is possible.

## Claims

1. A method of obtaining cell nuclei of foetal nucleated cells from a biological fluid containing at least 2 different nucleated cell populations, which comprises treating a sample of biological fluid suspected of containing foetal nucleated cells with a lytic reagent so as to selectively lyse the plasma membranes but not the nuclear membranes of the foetal nucleated cells whilst leaving 90-100% of the other nucleated cells intact, and wherein the foetal cell nuclei are separated from the other nucleated cells.

2. The method of claim 1 where the foetal nucleated cells are rare cells.

3. The method of claims 1 or 2 where the biological fluid is peripheral blood, cord blood, foetal blood or amniotic fluid from a pregnant woman.

4. The method of claims 1 to 3 where the lytic reagent contains a plant glycoside, detergent, inorganic salt or combinations thereof.

5. The method of claim 4 where the lytic reagent contains saponin.

6. The method of claim 5, wherein the lytic reagent comprises 0.01 - 0.1 wt% saponin and 10 - 100 mM alkali metal salt buffered at a pH of 7.0 - 7.5.

7. The method of claims 1 to 6 where the foetal cell nuclei are separated by attachment to a surface.

8. The method of claim 7 where the surface containing the foetal cell nuclei is washed to separate other intact cells or cell components.

9. An in-vitro method of identifying DNA from foetal nucleated cells when these cells are in admixture with other nucleated cells in a biological fluid sample which method comprises treating the mixture of nucleated cells with a lytic reagent so as to selectively lyse the plasma membranes but not the nuclear membranes of the foetal nucleated cells whilst leaving 90-100% of the other nucleated cells intact, separating the foetal cell nuclei and, optionally, lysing the foetal cell nuclei, followed by analysis of the foetal cell nuclei.

## Patentansprüche

1. Verfahren zum Gewinnen von Zellkernen aus fetalen, kernhaltigen Zellen aus einer biologischen Flüssigkeit, enthaltend mindestens zwei unterschiedliche kernhaltige Zellpopulationen, wobei das Verfahren die Behandlung einer Probe einer biologischen Flüssigkeit, in der fetale, kernhaltige Zellen vermutet werden, mit einem Lysemittel umfasst, so dass selektiv die Plasmamembranen, nicht jedoch die Kernmembranen der fetalen, kernhaltigen Zellen lysiert werden, während 90-100% der anderen kernhaltigen Zellen intakt bleiben, und wobei die fetalen Zellkerne von den anderen kernhaltigen Zellen abgetrennt werden.

2. Verfahren nach Anspruch 1, wobei die fetalen, kernhaltigen Zellen seltene Zellen sind.

3. Verfahren nach den Ansprüchen 1 oder 2, wobei die biologische Flüssigkeit peripheres Blut ist. Nabelschnurblut, fetales Blut oder Amnionflüssigkeit einer schwangeren Frau.

4. Verfahren nach den Ansprüchen 1 bis 3, wobei das Lysemittel ein Pflanzenglykosid, Detergens, anorganisches Salz oder Kombinationen daraus enthält.

5. Verfahren nach Anspruch 4, wobei das Lysemittel Saponin enthält.

6. Verfahren nach Anspruch 5, wobei das Lysemittel 0,01-0,1 Gew.-% Saponin und 10-100 mM Alkalimetallsalz, gepuffert bei einem pH von 7,0-7,5, umfasst.

7. Verfahren nach den Ansprüchen 1 bis 6, wobei die fetalen Zellkerne durch Anlagerung an eine Oberfläche abgetrennt werden.

8. Verfahren nach Anspruch 7, wobei die Oberfläche, die die fetalen Zellkerne enthält, gewaschen wird, um andere intakte Zellen oder Zellkomponenten abzutrennen.

9. In-vitro-Verfahren zur Identifizierung von DNA aus fetalen, kernhaltigen Zellen, wenn sich diese Zellen in einer Mischung mit anderen kernhaltigen Zellen in einer Probe einer biologischen Flüssigkeit befinden, wobei das Verfahren die Behandlung der Mischung der kernhaltigen Zellen mit einem Lysemittel, so dass selektiv die Plasmamembranen, nicht jedoch die Kernmembranen der fetalen, kernhaltigen Zellen lysiert werden, während 90-100% der anderen kernhaltigen Zellen intakt bleiben, die Abtrennung der fetalen Zellkerne und, wahlweise, die Lyse der fetalen Zellkerne, gefolgt durch Analyse der fetalen Zellkerne, umfasst.

## Revendications

1. Procédé d'obtention de noyaux cellulaires de cellules nucléées foetales à partir d'un fluide biologique contenant au moins deux populations de cellules nucléées différentes, qui comprend le fait de traiter un échantillon de fluide biologique soupçonné de contenir des cellules nucléées foetales avec un réactif lytique de façon à lyser sélectivement les membranes plasmiques mais pas les membranes nucléaires des cellules nucléées foetales, en laissant 90 à 100 % des autres cellules nucléées intactes, et dans lequel les noyaux de cellules foetales sont séparés des autres cellules nucléées.

2. Procédé de la revendication 1, dans lequel les cellules nucléées foetales sont des cellules rares.

3. Procédé des revendications 1 ou 2, dans lequel le fluide biologique est du sang périphérique, du sang de cordon, du sang foetal ou du liquide amniotique d'une femme enceinte.

4. Procédé des revendications 1 à 3, dans lequel le réactif lytique contient un glucoside de plante, un détergent, un sel inorganique ou des combinaisons de ceux-ci.

5. Procédé de la revendication 4, dans lequel le réactif lytique contient de la saponine.

6. Procédé de la revendication 5, dans lequel le réactif lytique comprend 0,01 à 0,1 % en poids de saponine et 10 à 100 mM de sels de métaux alcalins tamponnés à pH 7,0 - 7,5.

7. Procédé des revendications 1 à 6, dans lequel les noyaux de cellules foetales sont séparés par attachement à une surface.

8. Procédé de la revendication 7, dans lequel la surface contenant les noyaux de cellules foetales est lavée pour séparer d'autres cellules intactes ou composés cellulaires.

9. Procédé *in vitro* d'identification d'ADN à partir de cellules nucléées foetales lorsque ces cellules sont en mélange avec d'autres cellules nucléées dans un échantillon de fluide biologique, lequel procédé comprend le fait de traiter le mélange de cellules nucléées avec un réactif lytique de façon à lyser sélectivement les membranes plasmiques mais pas les membranes nucléaires des cellules nucléées foetales en laissant 90 à 100 % des autres cellules nucléées intactes, de séparer les noyaux des cellules foetales et, éventuellement, de lyser les noyaux de cellules foetales, suivie par une analyse des noyaux de cellules foetales.
